Europäisches Patentamt

European Patent Office

Office européen des brevets

(11) Numéro de publication: **0 252 787**
A1

# (12) DEMANDE DE BREVET EUROPEEN

(21) Numéro de dépôt: 87401331.1

(22) Date de dépôt: 12.06.87

(51) Int. Cl.4: **G 01 N 33/68**
G 01 N 33/564, C 07 K 15/06,
C 07 K 3/02

(30) Priorité: 12.06.86 FR 8608511   31.12.86 US 948253

(43) Date de publication de la demande:
13.01.88 Bulletin 88/02

(84) Etats contractants désignés:
AT BE CH DE ES FR GB GR IT LI LU NL SE

(71) Demandeur: INSTITUT PASTEUR
25-28, rue du Docteur Roux
F-75724 Paris Cédex 15 (FR)

INSTITUT NATIONAL DE LA SANTE ET DE LA
RECHERCHE MEDICALE (INSERM)
101, rue de Tolbiac
F-75654 Paris Cédex 13 (FR)

(72) Inventeur: Jacob, Laurent
37, rue Barbet de Jouy
F-75007 Paris (FR)

Lety, Marie-Annick
4 rue Edouard Detaille
F-92100 Boulogne (FR)

Bach, Jean-François
180, rue de Grenelle
F-75007 Paris (FR)

Louvard, Daniel
23 Allée de Trévise
F-92230 Sceaux (FR)

(74) Mandataire: Gutmann, Ernest et al
S.C. Ernest Gutmann - Yves Plasseraud 67, boulevard Haussmann
F-75008 Paris (FR)

Le (Les) microorganisme(s) a (ont) été déposé(s) auprès Collection Nationale de Cultures de Microorganismes sous le(s) numéro(s) I-555.

(54) Polypeptides de surfaces cellulaires, isolés et purifiés, reconnus par les anticorps responsables de la pathogénie du lupus érythémateux disséminé (LED), leur méthode d'obtention et leur application au diagnostic du LED.

(57) L'invention concerne des polypeptides de surfaces cellulaires situés à la surface de différentes lignées cellulaires humaines affectées par le LED, caractérisés notamment en ce qu'ils sont reconnus par des sérums de souris lupiques et de patients humains affectés par le LED.

L'invention concerne également un procédé d'obtention de ces polypeptides, ainsi que leur utilisation pour le diagnostic in vitro du LED.

EP 0 252 787 A1

**Description**

**POLYPEPTIDES DE SURFACES CELLULAIRES, ISOLES ET PURIFIES, RECONNUS PAR LES ANTICORPS RESPONSABLES DE LA PATHOGENIE DU LUPUS ERYTHEMATEUX DISSEMINE (LED) : LEUR METHODE D'OBTENTION ET LEUR APPLICATION AU DIAGNOSTIC DU LED**

L'invention concerne ces polypeptides de surface [lupus associated membrane protein(s) ou LAMP] qui partagent un épitope avec l'ADN, ainsi qu'un procédé de détection d'anticorps dirigés contre lesdits polypeptides et présents dans le sérum de ces patients atteints de lupus éryhtémateux disséminé (LED).

Le LED est une maladie auto-immune caractérisée par un dérèglement du système immunitaire aboutissant à la production d'anticorps anit-ADN double brin, responsables de lésions tissulaires.

Ainsi, la plupart des lésions tissulaires observées dans les cas de LED humains étaient considérées jusqu'à la réalisation de la présente invention comme étant dues à la formation de complexes immuns ADN-anti-ADN et à leurs dépôts au niveau des reins et d'autres tissus, bien que la présence d'ADN au sein de ces complexes immuns n'ait jamais pu être formellement démontrée et que l'on n'ait guère réussi jusqu'à ce jour à induire in vivo des anticorps contre des ADN utilisés en tant qu'antigènes dans des essais d'immunisation chez l'animal, notamment le lapin.

Il n'en reste pas moins que la présence de titres très élevés d'anticorps dirigés contre l'ADN double brin dans les sérums d'individus atteints de LED a jusqu'à ce jour été considérée comme un marqueur caractéristique du LED.

Les souris auto-immunes chez lesquelles la maladie peut se développer de la même manière que chez l'homme, constituent un bon modèle pour l'étude des mécanismes immunitaires présumés être responsable du LED. En particulier, les souris auto-immunes B/W et MRL/I présentent un LED identique au LED humain. Le sérum des patients atteints de LED contient un mélange d'anticorps dirigés contre des acides nucléiques variés. Il était donc légitime d'utiliser la technique des hybridomes pour mieux caractériser la spécificté des anticorps anti-ADN.

On a été amené à distinguer les anticorps dirigés contre l'ADN simple brin et les anticorps dirigés contre l'ADN double brin.

Cette distinction qui a connu un certain succès par les corrélations cliniques auxquelles elle a donné lieu, s'est rapidement révélée peu satisfaisante car il est techniquement difficile de caractériser l'ADN en termes d'ADN simple brin ou d'ADN double brin.

Il a en outre été constaté que les sérums de malades atteints de LED contiennent un mélange d'anticorps respectivement capables de réagir, non seulement avec l'ADN mais aussi avec l'ARN (acides ribonucléiques) et des nucléoprotéines. Il devenait difficile d'apprécier la signification individuelle d'un anticorps donné, dès lors que l'antigène correspondant n'était pas parfaitement défini.

Les obstacles à l'analyse fine des anticorps anti-ADN produits spontanément chez des souris lupiques ont été récemment levés par la production d'hybridomes synthétisant des anticorps monoclonaux anti-ADN simple brin (J. Immunol., 1980, 124, 1499-1502) et contre l'ADN double brin (J. Immunol., 1982, 128, 895-898).

Les expérimentations n'ont pas non seulement montré que ces titres ne pouvaient pas être corrélés aux normes révisées d'appréciation de l'évolution clinique de la maladie, publiée par l'American Rheumatism Association Tan EM Cohen AS, Fries J.F. et al (1982), the revised criteria for the classification of systemic lupus erythematus ; elles ont encore révélé que certains malades présentant les signes cliniques du LED n'étaient pas porteurs d'anticorps anti-ADN.

Au demeurant, la mise en jeu des ADN dans la formation des complexes immuns ADN-anti-ADN antérieurement envisagés était déjà sujette à caution.

C'est ainsi qu'il avait déjà été reconnu que des anticorps monoclonaux spécifiquement dirigés contre l'ADN double brin et obtenus à partir de souris autoimmunes B/W, se fixaient à la surface de cellules lymphoblastoïdes B humains de Raji, et que cette liaison n'était pas perturbée par un traitement de ces cellules avec l'ADNase (Eur. J. Immunol. , 14, 283-286, 1984). Il a également été montré, qu'une catégorie particulière d'anticorps monoclonaux (AcM) dirigés contre des ADN double brin, à titre d'exemple l'hybridome PME77, reconnaissaient des polypeptites à la surface des différentes lignées cellulaires humaines affectées par le LED : glomérules, lymphocytes B et T, érythocytes, plaquettes et système nerveux central [Proc. Natl. Acad. Sci. USA, 81, 3843-3845 (1984)] Des cellules de l'intestin, du pancréas et du foie, rarement affectés par le LED ne sont pas reconnues par l'anticorps produit par l'hybridome PME77.

Ces constatations découlaient, en effet, de ce que les cellules concernées n'étaient plus reconnues par l'AcM PME77 lorsqu'elles avaient été traitées par la protéinase K, d'où la conclusion que les polypeptides reconnus par ces anticorps étaient présents à la surface des cellules.

En outre, l'analyse de surnageant avait montré également que celui-ci contenait, en mélange avec un nombre considérable d'autres protéines, 5 polypeptides reconnaissables par les AcM PME77 après électrophorèse sur gel et immunotransferts.Leurs poids moléculaires devaient être respectivement de l'ordre de 34 000, 33 000, 17 000, 16 000 et 14 000.

Ces résultats suggéraient qu'il existe un déterminant antigénique commun entre ces polypeptides présents à la surface des cellules de Raji et l'ADN double brin. Ces polypeptides sont également présents à la surface des différents types cellulaires concernés dans la pathogénie du LED.

La présente invention découle précisément de la découverte des conditions dans lesquelles il devenait possible d'isoler des polypeptides susceptibles d'être reconnus par cet AcM PME77 à la surface des cellules, et ce dans des conditions de pureté telles que des compositions douées de propriétés nouvelles ont été obtenues. En particulier, il a été découvert que, dans cet état de pureté, ces polypeptides étaient reconnus, non seulement par le précédent anticorps, mais aussi par les anticorps présents dans les sérums d'individus atteints de LED. En particulier, l'invention concerne une composition de polypeptides correspondant à une protéine avec ses produits de dégradation, ou à plusieurs protéines.

Ces polypeptides sont caractérisés :
- en ce qu'ils sont spécifiquement reconnus par l'AcM PME77 ou un AcM équivalent.
- en ce qu'ils sont présents dans cette composition à une concentration suffisamment élevée pour autoriser leur fixation sur l'AcM PME77 lui-même immobilisé sur un support insoluble dans des opérations de chromatographie d'affinité et en ce que
- ces polypeptides sont reconnus par des sérums de souris lupiques et de patients humains affectés par le lupus érythémateux disséminé (LED) et non reconnus par des sérums obtenus à partir de personnes saines ou de souris saines,
- ces polypeptides sont immunogènes chez le lapin et induisent des anticorps qui ne reconnaissent pas l'ADN double brin,
- ces polypeptides sont reconnus par des immunoglobulines éluées de reins de souris lupiques et ne sont pas reconnus par des immunoglobulines éluées de reins de souris non lupiques.

Il est entendu que sous l'expression "polypeptide" reconnu par l'AcM PME77, on entend toutes molécules, qu'il s'agisse d'une protéine, d'un polypeptide vrai (chaîne d'amino acides) ou un fragment de protéine ou un peptide ou encore des molécules comprenant d'autres groupes fixés sur le polypeptide, par exemple des glycoprotéines ou lipoprotéines ou autre ligand.

L'invention concerne également un procédé permettant d'obtenir les susdits polypeptides. Ce procédé comprend une étape d'incubation ménagée des cellules susceptibles de porter à leur surface les polypeptides concernés par l'invention en présence d'une enzyme du type élastase, la récupération du surnageant contenant les polypeptides de surface reconnaissables par l'AcM PME77 et, le cas échéant, la purification desdits polypeptides.

En d'autres termes, l'invention concerne un procédé d'obtention d'une composition de polypeptides telle qu'elle a été définie ci-dessus, qui comprend la mise en incubation de cellules humaines ou de cellules de souris ou de rat dans une solution contenant de l'élastase en concentration dosée et dans des conditions de pH, température et temps réglées de façon à permettre une extraction sélective desdits polypeptides à partir de la surface desdites cellules, puis la récupération du surnageant contenant les polypeptides reconnaissables par l'anticorps monoclonal AcM PME77.

En particulier, l'incubation des cellules est réalisée en présence d'élastase à une concentration finale de 5 à 20 µg/ml notamment de 10 µg/ml, pendant une durée de 5 à 15 minutes, notamment 7 mn, à une température de 4° dans une solution tamponnée à pH 8,2 (compris entre 8 à 8,5).

N'importe quel type de cellules provenant de l'homme, de la souris ou du rat peut être mis en oeuvre dans le procédé selon l'invention.

De préférence, les cellules en cause seront choisies parmi celles qui peuvent être cultivées et qui présentent des teneurs élevées en protéine à leur surface, par exemple des cellules indifférenciées.

Il va de soi que les différents paramètres de l'incubation ménagée desdites cellules en présence de l'elastase pourront éventuellement être modifiés.

Il est clair que l'homme de métier, disposant de la connaissance que lui apporte la présente demande de brevet, sera capable de déterminer les conditions les plus favorables pour régler les conditions de la protéolyse ménagée par l'élastase, en vue d'obtenir les meilleurs rendements en élastase, pour la récupération des polypeptides recherchés à partir d'un type donné de cultures cellulaires.

La possibilité d'isoler ces polypeptides répondant aux conditions définies ci-dessus, par l'intermédiaire d'un traitement ménagé des cellules par l'élastase était d'autant plus remarquable qu'un tel résultat s'est révélé inaccessible avec un grand nombre d'autres protéases.

Les compositions selon l'invention, en particulier le "surnageant élastase" dans les conditions sus-indiquées, peuvent être davantage purifiés.

De préférence, un prodédé de purification similaire comprend la mise en contact de ce surnageant avec l'anticorps monoclonal AcM PME77 (ou des anticorps monoclonaux ou polyclonaux préalablement formés contre ces polypeptides) immobilisé sur un support de chormatographie d'affinité, et la récupération des polypeptides retenus par la mise en contact du support de chromatographie d'affinité avec une solution de dissociation du complexe antigène-anticorps formé.

En d'autres termes, un procédé préféré de purification de l'invention comprend les étapes consistant à faire passer une solution contenant ces polypeptides au contact d'une colonne d'affinité portant les susdits anticorps immobilisés sur un support adéquat (par exemple le réseau d'agarose à réticulation tridimensionnelle commercialisé sous la désignation SEPHAROSE 4B par la société suédoise PHARMACIA A.G.) pour fixer sélectivement lesdits polypeptides, puis à récupérer ceux-ci par dissociation du complexe antigène-anticorps formé sur la colonne au moyen d'un tampon approprié, ayant notamment une force ionique adéquate. Un tampon approprié consiste par exemple en une solution d'un sel, de préférence l'acétate d'ammonium (lequel ne laisse pas de résidu lorsque l'on réalise ensuite la lyophilisation de la préparation). On peut également avoir recours à une solution acidifié à pH 3-4 ou à un tampon glycine au même pH. D'autres

3

méthodes de purification peuvent être utilisées.

On a déterminé par élécrotphorèse et analyse sur immunotranfert (immunoempreinte) que ce surnageant contient au moins 5 polypeptides majeurs dont les poids moléculaires sont comme précédemment de l'ordre (à plus ou moins 10 % près) de 34 KD, 33 KD, 17 KD, 16 KD et 14 KD, tous porteurs d'épitopes reconnus par les AcM PME77. En particulier, l'AcM PME77 reconnait également un polypeptide dont le poids moléculaire est de l'ordre de 50 KD, voire même de 55 KD. Ce dernier polypeptide peut, en particulier, être obtenu à partir de cellules de rate de souris lupiques traitées à l'élastase. L'invention concerne plus particulièrement un extrait purifié de polypeptides reconnus par l'AcM PME77, selon la méthode précédemment décrite, et composé essentiellement de plusieurs espèces moléculaires, tous reconnus par l'AcM PME77.

Naturellement, ces polypeptides peuvent être séparés les uns des autres par exemple par électrophorèse sur gel approprié et récupération des différentes bandes à partir de ce gel.

L'invention concerne également les anticorps dirigés contre les polypeptides de 17 KD, 16 KD et 14 KD, par exemple ceux produits par immunisation de lapins, à l'aide d'un extrait purifié, selon l'invention, composé de ces 3 polypeptides.

Le sérum de ces lapins immunisés ou des préparations davantage purifiées de ces anticorps seront, dans ce qui suit, désignés sous l'expression "anticorps polyclonal".

Les inventeurs ont démontré, par analyse d'immutransfert obtenu à partir d'un extrait résultant du traitement de cellules IW 32 de souris avec de l'élastase dans des conditions douces selon la méthode précedemment décrite, que cet "anticorps polyclonal" reconnait les 4 polypeptides majeurs de 43 KD, 17 KD, 16 KD et 14 KD.

Cet anticorps polyclonal a été purifié par récupération de bandes d'électrophorèse correspondant au polypeptide 17 KD (qui est le mieux reconnu par l'anticorps polyclonal) sur laquelle il s'est fixé et qui ont été traitées selon la méthode décrite par Coudrier et al (Embo. J.,2, 469-475, 1983).

Le fait que cet anticorps polyclonal purifié par affinité, dirigé contre le polypeptide de 17 KD isolé, réagit avec celui de 34 KD et celui de 43 KD, après immunoaffinité, souligne la réactivité croisée existante entre ces différentes espèces de polypeptides.

Il n'est pas exclu que les différents polypeptides qui se distinguent les uns des autres par des poids moléculaires distincts soient des fragments résultant de la protéolyse d'un polypeptide plus grand, par exemple possédant un poids moléculaire de 43 KD, voire davantage.

D'une façon générale, l'invention ne saurait être limitée à des compositions ne contenant que les polypeptides présentant les poids moléculaires spécifiques mentionnés ci-dessus. Tout polypeptide pourvu d'un site ou déterminant antigénique ou épitope reconnu par l'AcM PME77, fait partie des "polypeptides" libres entrant dans le cadre de l'invention.

Par "libre", il faut entendre un polypeptide détaché de son support moléculaire et en concentration suffisante dans les compositions qui le contiennent, pour être reconnu par l'AcM PME77 immobilisé sur un support de chromatographie d'affinité.

Il est important, en outre, de remarquer que l'anticorps polyclonal ne réagit pas avec l'ADN double brin, démontrant ainsi ques les épitopes majeurs de ces polypeptides, ou du polypeptide unique, dont ils sont éventuellement dérivés, ne partagent pas d'épitope avec l'ADN double brin.

Une telle découverte a permis aux inventeurs de suggérer que ces polypeptides de surface cellulaire (LAMP) doivent agir en tant qu'immunogènes potentiels lorsque l'on rencontre des situations physiopathologiques associées au développement du LED, les ADN seraient alors hors de cause.

Une autre observation vient appuyer cette hypothèse. En effet, les immunoglobulines éluées à partir de reins de souris lupiques, notamment de souris auto-immunes MRL/lpr réagissent spécifiquement avec ce ou ces polypeptides.

Cette découverte tend à prouver que ce ou ces polypeptides (LAMP) pourraient jouer un rôle dans les lésions tissulaires rénales observées dans le LED murin, et intervenir dans la production de complexes antigènes-anticorps dans lesquels interviennent vraisemblablement les grandes quantités d'anticorps présents dans les reins de souris lupiques.

Egalement en faveur d'une telle hypothèse, on peut rappeler que la présence d'ADN dans les complexes immuns ADN-anti-ADN n'a jamais été formellement démontrée.

Enfin, l'invention met à profit la propriété de ces polypeptides de surface à être spécifiquement reconnus par les anticorps présents dans les sérums humains d'individus atteints de LED, dans des essais de diagnostic in vitro du LED.

Les inventeurs ont en effet découvert que la totalité des sérums humains testés, dilués au 1 : 20, provenant du groupe des 25 malades atteints de LED dont il est question plus haut, contiennent des anticorps qui reconnaissent les polypeptides présents dans le "surnageant d'élastase" précédemment décrit.

L'invention concerne également de façon générale un procédé de détection in vitro d'anticorps corrélables au lupus érythémateux disséminé (LED) dans un tissu ou fluide biologique susceptible de la contenir, ce procédé comprenant la mise en contact de ce tissu ou fluide biologique avec un composition contenant le polypeptide reconnu par l'AcM PME77 dans des conditions permettant une réaction immunologique in vitro entre lesdits polypeptides et les anticorps éventuellement présents dans le tissu ou fluide biologique, et la détection in vitro du complexe antigène-anticorps éventuellement formé.

De préférence, le milieu biologique est constitué par un sérum humain.

Toute procédure classique peut être mis en oeuvre pour réaliser une telle détection.

A titre d'exemple une méthode préférée met en jeu des procéssus immunoenzymatiques selon la technique ELISA, ou immunofluorescents, ou radioimmunologiques (RIA) ou équivalent.

Ainsi l'invention concerne également tout polypeptide selon l'invention marqué à l'aide d'un marqueur adéquat du type enzymatique, fluorescent, radioactif, etc...

De telles méthodes comprennent par exemple les étapes suivantes :
- dépôt de quantités déterminées da la composition polypeptidique selon l'invention dans les puits d'une microplaque de titration,
- introduction dans lesdits puits de dilutions croissantes du sérum devant être diagnostiqué,
- incubation de la microplaque,
- rinçages répétés de la microplaque,
- introduction dans les puits de la microplaque d'anticorps marqués contre des immunogobulines du sang, le marquage de ces anticorps ayant été réalisé à l'aide d'une enzyme sélectionnée parmi celles qui sont capables d'hydrolyser un substrat en modifiant l'absorption des radiations de ce dernier, au moins à une longueur d'onde déterminée,
- détection, en comparaison avec un témoin de contrôle, de la quantité de substrat hydrolysé.

L'invention concerne également des coffrets ou kits pour le diagnostic in vitro du LED qui comprennent :
- une composition contenant au moins un polypeptide (marqué ou non) reconnu par l'AcM PME77,
- les réactifs pour la constitution du milieu propice à la réalisation de la réaction immunologique,
- les réactifs permettant la détection du complexe antigènes-anticorps produit par la réaction immunologique. De tels réactifs peuvent également porter un marqueur, ou être susceptibles d'être reconnus à leur tour par un réactif marqué, plus particulièrement dans le cas où la composition polypeptidique sus-mentionnée n'est pas marquée.
- un tissu fluide biologique de référence dépouvu d'anticorps reconnus par la composition polypeptide sus-mentionnée.

L'invention concerne les anticorps eux-même formés contre les polypeptides de l'invention.

Il va de soi que cette production n'est pas limitée aux anticorps polyclonaux.

Elle s'applique encore à tout anticorps monoclonal produit par tout hybridome susceptible d'être formé, par des méthodes classiques, à partir des cellules spléniques d'un animal, notamment de souris ou de rat, immunisés contre l'un des polypeptides purifiés de l'invention, d'une part et des cellules d'une lignée de cellule myélome approprié d'autre part, et d'être sélectionné, par se capacité à produire des anticorps monoclonaux reconnaissant le polypeptide initialement mis en oeuvre pour l'immunisation des animaux.

Les polypeptides du genre en question peuvent être également utilisés pour séparer des anticorps présentant les caractéristiques sus-indiquées à partir d'un mélange d'immunoglobulines contenant ces anticorps. Dans ce cas, les polypeptides du genre en question seront à leur tour immobilisés sur un support de chromatographie d'affinité, par exemple du genre indiqué plus haut. Le procédé de séparation comprend par conséquent l'étape consistant à faire passer une solution contenant les anticorps polyclonaux au contact des polypeptides immobilisés, et l'étape de récupération des anticorps retenus au moyen d'une solution ou d'un tampon analogue à celle ou celui qui a été envisagé plus haut.

Enfin, l'invention concerne des compositions immunogènes caractérisées par l'association de l'un des antigènes immunogènes sus-indiqués avec un excipient physiologiquement acceptable permettant leur administration à un hôte vivant, en vue de lui conférer une immunité à l'égard desdits polypeptides. Ces polypeptides constituent des principes actifs dont l'immunogénicité peut être mise en oeuvre à chaque fois qu'est recherchée une protection in vivo contre eux-mêmes.

Il va de soi que l'invention concerne également des fractions polypeptidiques pouvant avoir des poids moléculaires plus faibles, dès lors qu'ils porteraient des sites antigéniques susceptibles d'être reconnus par les mêmes anticorps monoclonaux. Il apparaîtra clairement au spécialiste qu'à partir de l'instant où l'on dispose des anticorps monoclonaux considérés ci-dessus, on peut envisager l'isolement, à partir des antigènes sus-indiqués, des séquences peptidiques plus petites contenant les mêmes sites antigéniques, par exemple en ayant recours à des techniques en soi connues de découpage des polypeptides par des enzyme susceptibles de découper les polypeptides plus grands en des sites spécifiques. A titre d'exemple de telles protéines, on peut mentionner l'enzyme de Staphylococcus aureus V8, l'alpha-chymotrypsine, la "protéase de glandes sous-maxillaires de souris" (mouse submaxillary gland protease) commercialisée par la société BOEHRINGER, la collagénase de Vibrio alginolyticus chemovar iophagus qui reconnait spécifiquement lesdits peptides Gly-Pro et Gly-Ala, etc. Les plus petits fragments de peptides reconnus par l'AcM PME77 peuvent donc être isolés, caractérisés et synthétisés.

D'autre part, après détermination de la séquence peptidique des polypeptides de l'invention, il devient possible de reconstituer des séquences nucléotidiques correspondantes ; ces dernières peuvent à leur tour être synthétisées et clonées.

Des caractéristiques supplémentaires de l'invention apparaîtront encore au cours de la description des techniques et conditions dans lesquelles des polypeptides conformes à l'invention ont été obtenus.

I. Isolement et purification des polypeptides de surface cellulaire

## 1) Matériel et méthodes

Les marqueurs des poids moléculaires sont les suivants : phosphorylase b (93 000), sérum albumine bovine (66 000), ovalbumine (43 000), anhydrase carbonique (30 000), inhibiteur de la trypsine (20 000), et lactalbumine (14 000).

### - Sérum

Les sérums ont été obtenus à partir de 25 individus atteints de LED. Ces sérums ont été chauffés pendant 30 mn à 56°C afin d'inactiver le complément et ont été conservés à -20°C. Dix sérums d'humains normaux (SHN) ont été utilisés comme contrôles.

### - Lignées cellulaires

Les lignées cellulaires lymphoblastoïdes B de Raji et des cellules de souris érythroleucémiques IW 32 ont été obtenues et maintenues comme précédemment décrit dans Eur. J. Immunol. , 14, 283-286, 1984.

### - AcM murin

Le surnageant d'hybridomes PME 77, secrétant un anticorps anti-ADN, a été isolé après fusion entre des cellules d'un myélome non secréteur (P3 x 63 Ag 8653) et des cellules de rate de (NZB x NZW) f1. L'AcM PME77 a été décrit comme étant une immunoglobuline G2b, un anticorps à chaîne K et spécifique de l'ADN [J. Immunol., 125, 2805-2809 (1980), et J. Immunol., 128, 895-898, (1982)]

L'hybridome secréteur de l'AcM PME77 a été déposé à la C.N.C.M le 15 mai 1986 sous le numéro I-555.

### - Traitement des cellules Raji et des cellules IW 32 avec l'élastase

Dix millions de cellules Raji et IW 32 viables ont été cultivées en milieu RPMI avec 200 µCi de $^{35}$S Methionine pendant 4 heures.

Après cette période, les cellules ont été centrifugées à 200 x g à 4°C et resuspendues dans 1 ml d'élastase (concentration finale 10 µ/ml) (Merck) dans une solution tampon contenant du Tris 50 mM, Nacl 0,15 M, pH 8,0 pendant 7 mn, et à froid (bain-marie glacé).

Les cellules ont été séparées des protéines de surface, relarguées dans le milieu par l'élastase, par centrifugation à vitesse lente (200 x g, pendant 7 mn). Le "surnageant élastase" a été gardé et analysé.

### - Analyse des immunotransferts du "surnageant élastase"

Un aliquot de "surnageant élastase" a été soumis à une électrophorèse sur gel de polyacrylamide de sodium dodecyl sulfate (SDS) selon Laemmli (Nature (Lond), 227, 680-684).

Après électrophorèse, la réalisation des immunotransferts a été effectuée selon la méthode décrite par Burnette (anal. Biochem. 1981, 112, 195-203) et modifiée par Coudrier et al [Embo. J., 2, 469-475, (1983)].

### - analyse par immunoprécipitation du "surnageant élastase"

Le "surnageant élastase" a été incubé en présence d'AcM PME77 pendant 18 heures à 4°C.

Les complexes immuns ont été précipités avec des anticorps de lapin anti-souris (20 µg) pendant 30 mn à température ambiante ainsi que, dans les mêmes conditions, avec de la proteine A fixée sur le support commercialisé sous la désignation SEPHAROSE 4B (Pharmacia) (ci-après dénommée "SEPHAROSE 4B-Protéine A"), 40 µg.

Les échantillons ont été lavés 7 fois dans une solution tampon contenant du Nacl 190 mM, Tris 50 mM, EDTA 6 mM, Triton X-100 (2,5 % V/V) à pH 7,4 et soumis à une électrophorèse selon la méthode de Laemmli.

Le gel a été trempé dans un amplificateur (NEN) pour fluorographie. Le gel séché a été exposé à un film Kodak ARX5 à - 80°C pendant 24 heures.

### - Purification des polypeptides de surface cellulaire à partir des cellules IW 32

#### . Préparation de l'immunoabsorbant

Le surnageant de l'hybridome secrétant l'anticorps anti-ADN PME77 a été passé à travers une colonne de SEPHAROSE 4B Protéine A. Puis l'AcM a été lié de façon covalente à la Protéine A par l'intermédiaire du Dimethyl suberimidate (DMS) ("agent de réticulation") comme décrit par Coudrier et al (J. Mol. Biol., 152, 49-66).

#### . Procédé de purification

Le "surnageant d'élastase" a été passé à travers une colonne de SEPHAROSE 4B Protéine A liée à l'AcM PME77.

La colonne a été lavée avec une solution de PBS ajustée à pH 7,4 et de NaCl 0,5 M en présence de Triton X-100 (0,1 % V/V).

Les polypeptides ont été élués dans une solution tampon comprenant HCl 0,2 M, ajustée à pH 2,2 avec de la glycine.

L'éluat a été tamponné jusqu'à neutralité à l'aide de Tris-base (solution 1M).

- Electrophorèse sur gel et analyse par immunoprécipitation des polypeptides élués

Un aliquot des polypeptides élués marqués avec la methionine $^{35}$S a été soumis à une électrophorèse sur gel de polyacrylamide (PAGE) et fluorophotographié.

Dans une autre expérience, un aliquot de ces polypeptides a également été immunoprécipité avec l'AcM PME77.

- Préparation d'un anticorps polyclonal de lapin dirigé contre les polypeptides de surface cellulaire

Un lapin a été immunisé avec les polypeptides de surface cellulaire purifiés utilisés en tant qu'antigènes absorbés sur nitrocellulose. Trois polypeptides de 17 KD, 16 KD et 14 KD ont été visualisés sur feuille de nitrocellulose avec du PONCEAU S selon Coudrier et al (Embo. J. précédemment cité).

Les morceaux de nitrocellulose ont alors été trempés dans l'azote liquide et écrasés à l'aide d'un pilon dans un mortier refroidi dans l'azote liquide ; la nitrocellulose pulvérisée a été resuspendue dans 500 μl d'une solution normale de chlorure de sodium et émulsifiée avec 500 μl d'adjuvant complet de Freund (CFA, DIFCO, Detroit, MI).

Pour la première injection chez le lapin, le matériel a été injecté dans le ganglion lymphatique poplitéal (environ 5 μg) et dans le dos par voie intradermique le long de la colonne vertébrale (environ 5 μg) en

Le premier rappel a été donné 3 semaines plus tard avec la même dose d'antigène absorbé sur nitrocellulose pulvérisée, émulsifiée dans l'adjuvant incomplet de Freund (DIFCO) et administrée par injections sous-cutanées (5 μg) et intrapéritonéales (5 μg).

Le second rappel sous-cutané a été donné au 21e jour en utilisant l'antigène absorbé sur nitrocellulose et resuspendu dans le PBS.

Le premier prélèvement de sang a été réalisé une semaine après le troisième rappel puis, par la suite, une fois par semaine pendant au moins un mois.

- Expériences de fixation aux cellules de l'anticorps polyclonal de lapin.

Ces expériences ont été réalisées selon la tchnique décrite dans Euro. J. of Immunol. (1984), 14, 283-286, avec l'AcM PME77, l'anticorps polyclonal de lapin et le sérum non immun de lapin utilisé comme contrôle.

- Electrophorèse sur gel et analyse par immunoréplique de l'anticorps polyclonal de lapin et des sérums LED humains

Un extrait total de cellules IW 32 et un aliquot de "surnageant élastase" ont été préparés comme précédemment décrit.

Après électrotransfert des polypeptides, la feuille de nitrocellulose a été incubée avec l'anticorps polyclonal dilué au 1 : 100, et 25 sérums LED humains dilués au 1 : 50 pendant 90 mn à temperature ambiante.

Les complexes antigène-antisérum ont été détectés par les techniques à la peroxydase.

- Procédure de purification par affinité de l'anticorps polyclonal à partir de la bande spécifique de 17 KD

Une purification d'affinité à micro-échelle des anticorps a été réalisée à partir des bandes supportées.

Plusieurs échantillons d'extrait total de cellules IW 32 ont été soumis à l'analyse par immunotransfert comme précédemment décrit.

Les bands de 17 KD, spécifiquement reconnus par l'anticorps polyclonal, ont été précisément localisées sur le filtre. Chacune de ces bandes a été découpée et traitée comme précédemment décrit en détail par Coudrier et al (Embo. J. précédemment cité).

- Capacité de fixation à l'ADN de l'anticorps polyclonal

La capacité de fixation à l'ADN de l'anticorps polyclonal a été mesurée en utilisant un procédé radio-immunologique en phase solide comme préalablement décrit dans J. Immunol., 128, 895-898, (1982).

2) Résultats

- Les antigènes reconnus par l'AcM PME77 sont détachés des cellules Raji par l'élastase

De manière à déterminer plus précisément la nature des antigènes reconnus par l'AcM PME77 et pour les domaines accessibles à la surface des cellules, des traitements avec plusieurs protéases ont été mis en oeuvre. Ces antigènes se sont révélés très sensibles aux traitements à la papaine et à la trypsine.

Même lorsque les concentrations (10 μg/ml) de protéase étaient très faibles et les durées de traitement très courtes (5 mn) au froid (4°C), aucun fragment reconnaissable par l'AcM PME77 n'a pu être isolé à partir du surnageant.

Néanmoins, ces antigènes ont été détachés de la surface des cellules puisqu'ils n'ont pu être détectés dans le culot cellulaire après protéolyse.

Toutefois, lorsqu'un traitement doux à l'élastase a été utilisé, les fragments accessibles de la surface des cellules ont été libérés et détectés dans le surnageant. Au surplus, les plus grands fragments détectés par AcM PME77 ont pu être isolés efficacement à partir du surnageant quand l'élastase a été utilisé à pH 8,2 et à concentration de 10 μg/ml pendant 7 mn.

Ces résultats démontrent la sensibilité de ces polypeptides aux protéases.

Une famille de polypeptides, migrant le plus souvent à 34 KD, 33 KD, 17 KD, 16 KD et 14 KD, a été détectée dans le "surnageant élastase" avec l'AcM PME77 par analyse d'immunotransfert de polypeptides ("immunoblotting").

Toutefois, en raison d'une extrême sensibilité de ces polypeptides, quelques autres bandes ont pu être détectées en plus des bandes majeures en fonction des conditions expérimentales.

- Analyse du "surnageant élastase" par immunoprécipitation

Dans les expériences d'immunoprécipitation, en utilisant le "surnageant élastase", l'AcM PME77 immunoprécipite spécifiquement 3 polypeptides de 17 KD, 16 KD et 14 KD. L'absence de 2 polypeptides de 34 KD et 33 KD pourrait s'expliquer par la protéolyse de ces polypeptides en raison des longues périodes d'incubation et des nombreux lavages nécessaires dans cette technique.

En variante, les épitopes présents sur les polypeptides de 34 KD et 33 KD pourraient ne pas être accessibles sous la forme native de l'antigène mais seulement reconnus après dénaturation au SDS. En revanche, ces polypeptides n'ont pas été immunoprécipités par l'AcM anti-golgi utilisé en tant que témoins de contrôle [Embo. Journal (1982), 1, 1621-1628].

- Isolement des polypeptides de surface cellulaire

Au moins 4 polypeptides majeurs à 43 KD, 17 KD, 16 KD et 14 KD libérés de la surface des cellules IW 32 ont pu être isolés par chromatographie d'affinité avec, en tant qu'immunoadsorbant, l'AcM PME77 lié à la SEPHAROSE 4B Protéine A.

La pureté de ces polypeptides a été démontrée par électrophorèse d'un aliquot de polypeptides isolés sur un gel de polyacrylamide.

En revanche, lorsque dans la même expérience, l'AcM anti-Golgi lié à la SEPAHROSE 4B Protéine A a été utilisé comme contrôle, aucun des polypeptides n'a été piégé de façon non spécifique sur l'immunoabsorbant.

Dans une autre expérience, les polypeptides élués ont été immunoprécipités et analysés en utilisant l'AcM PME77 en solution.

Trois polypeptides à 17 KD, 16 KD et 14 KD ont été reconnus mais pas celui à 43 KD. Cette observation coincide encore avec la notion que le 43 KD est vraisemblablement dégradé en plus petits fragments durant la procédure d'immunoprécipitation.

Le polypeptide 50-55 KD est obtenu à partir du "surnageant élastase" préparé dans les mêmes conditions en utilisant des lymphocytes totaux de rates de souris lupiques MRL/lpr/lpr.

- Propriétés d'un anticorps polyclonal dirigé contre la (les) protéine(s) de surface cellulaire ayant un épitope commun avec l'ADN double brin (ADNdb)

Un antisérum a été produit en immunisant un lapin avec les 3 polypeptides immunologiquement apparentés de 17 KD, 16 KD, et 14 KD récupérés par immunoaffinité et par électrophorèse préparative sur gel de polyacrylamide et transfert sur filtre de nitro cellulose.

Des expériences de fixation de l'anticorps polyclonal et de la protéine A marquée à l'$^{125}$I sur les cellules, ont été réalisées.

Les résultats, résumés au tableau I, indique clairement l'existence d'une liaison spécifique aux cellules.

Au moins 4 bandes majeures peuvent être détectées à 43 KD, 17 KD, 16 KD et 14 KD par analyse d'immunotransfert avec une extrait total de cellules IW 32, en utilisant l'anticorps polyclonal.

Deux bandes à 43 KD et 17 KD présentent une forte réactivité, et les deux autres une plus faible réactivité.

Lorsqu'un sérum non-immun de lapin est utilisé à la même dilution en tant que témoin, aucune bande spécifique n'est détectée.

Afin de déterminer l'accessibilité à la surface cellulaire de l'antigène reconnu par l'anticorps polyclonal et de confirmer sa nature protéique, un traitement à l'élastase (100 µg/ml) a été réalisé sur des cellules vivantes.

Les quatre bandes ont alors pratiquement complètement disparues.

De manière à tester une éventuelle réaction croisée entre les polypeptides et l'ADNdb, l'anticorps polyclonal a été préincubé avec 1 mg/ml d'ADNdb. Les quatre bandes sont toujours observées avec la même intensité.

De plus, l'anticorps ne se lie pas à l'ADNdb dans un essai de détection radio-immunologique (RIA : abréviation de l'expression anglaise "radio-immuno-assay") (tableau II).

Ce résultat indique que l'anticorps polyclonal contient principalement des anticorps réagissant avec des épitopes ne reconnaissant pas l'ADNdb.

- Relation entre le polypeptide de 43 KD et celui de 17 KD reconnu par l'anticorps polyclonal

Une purification à micro-échelle par affinité de l'anticorps polyclonal à partir de la bande 17 KD a été réalisée comme précédemment décrit.

## TABLEAU I

Capacité de fixation de l'anticorps polyclonal de lapin
aux cellules de RAJI

| source de sérum | capacité de fixation |
|---|---|
| PBS | 228 ± 24 |
| ACM PME77 (100 µg/ml) | 8520 ± 529 |
| Anticorps polyclonal de lapin dilué au 1 : 100 | 5302 ± 412 |
| Anticorps polyclonal de lapin dilué au 1 : 500 | 2478 ± 223 |
| Sérum normal de lapin dilué au 1 : 100 | 1417 ± 221 |
| Sérum normal de lapin dilué au 1 : 500 | 567 ± 89 |

## TABLEAU II

<u>Capacité de fixation sur l'ADN, dans un essai radioimmunologique sur filtre d'ester de cellulose</u>.

| Source de sérum | fixation, cpm par plaque |
|---|---|
| Antisérum de lapin immunisé avec des protéines de surface de cellules, diluées au 1 : 10 | 157 ± 13 |
| Sérum normal de lapin dilué au 1 : 10 | 175 ± 22 |
| Surnageant d'AcM PME77 | 2355 ± 178 |
| Surnageant d'AcM anti-Golgi | 223 ± 64 |

L'anticorps purifié a été soumis à une analyse par immunotransfert sur un extrait total de cellules IW 32. Une famille de polypeptides de 43KD, 34 KD, 17 KD, 16 KD et 14 KD a été spécifiquement détectée suggérant la parenté antigénique existant entre les différents polypeptides. Un polypeptide de 23 KD, probablement dû à la variation des conditions expérimentales, a été observé dans cette expérience particulière.

- Analyse des immunotransferts de sérums LED humains

Trois bandes ont été détectées à 34 KD, 17 KD et 16 KD avec le sérum LED humain dilué au 1 : 50 en utilisant le "surnageant élastase" comme antigène.

Lorsque le sérum LED humain dilué au 1 : 50 a été préincubé avec l'ADNdb (1 mg/ml), l'intensité des 2 bandes de 34 KD et 17 KD a été fortement réduite alors que celle à 16 KD a été moins réduite.

Des résultats similaires ont été obtenus avec 5 autres sérums LED humains, démontrant clairement la présence d'anticorps dirigés contre ces polypeptides de surface cellulaire dans les sérums LED humains. Aucune bande n'a été observée lorsque 4 sérums humains normaux ont été utilisés comme contrôle.

II. Mise en évidence de la reconnaisance des polypeptides de surface cellulaire par des immunoglobulines obtenus à partir d'éluats de reins de souris autoimmunes MRL/lpr/lpr, et par des sérums de souris MRL/lpr et (NZB x NZW) F1 B/W

### 1. Matériel et méthodes

- Souris

Les souris MRL/lpr/lpr âgées de 16 semaines ont été obtenues à l'Institut Pasteur, (Paris, France).

Les souris B/W proviennent d'un croisement entre femelles NZB et mâles NZW et sont âgées de 6 mois.

Les souris NZB et BALB/c ont été fournies par le laboratoire CSEAL, du CNRS, (Orléans, la Source, France).

- Sérums

Les sérums des souris MRL/lpr, B/W, et BALB/c ont été chauffés pendant 30 mn à 56°C afin d'inactiver le complément, et stockés à -20°C.

Les polypeptides mis en jeu dans les essais qui suivent sont ceux du "surnageant élastase" envisagé plus haut, obtenu à partir des cellules de la lignée IW 32.

- Procédé d'élution des immunogobulines à partir de reins de souris autoimmunes MRL/lpr et BALB/c.

Huit souris MRL/lpr (souris lupiques) et huit souris BALB/c (souris saines)ont été perfusées avec 20 ml d'une solution solution de chlorure de sodium, et leurs reins ont été congelés dans l'azote liquide.

Seize reins de souris MRL/lpr et seize reins de souris BALB/c ont été respectivement regroupés, découpés en petits morceaux tout en les maintenant congelés, mis en suspension dans une solution tamponnée de phosphate de chlorure de sodium 0,15 M, pH 7,2, puis homogénéisés à 0°C.

L'homogénat a été lavé à 4°C avec 500 ml de PBS par centrifugation à 4000 x g. Ce procédé de lavage a été répété trois fois et le culot de centrifugation remis en suspension et incubé pendant 2 heures à 37°C dans 20 volumes de tampon citrate 0,02 M, pH 3,2 (J. Immunol. Methods (1982) 48 : 133).

Après élution, les tissus rénaux ont été centrifugés (4000 x g pendant 30 mn), et les produits d'élution ont été neutralisés à pH 7,0 avec du Na$_2$HPO$_4$ (1M), dialysés contre du PBS pendant une nuit, concentrés, ajustés à 2 ml et stockés à -20°C. La densité optique est approximativement de 3,7 pour MRL/lpr et 1,1 pour BALB/c.

- Test ELISA

Ce test de détection des IgG éluées à partir des reins a été réalisé de la manière décrite dans Proc. Natl. Acad. Sci. USA (1983) 80, 6269-6273.

Brièvement, sur des plaques de microtest commercialisé sous la désignation LUXLON (CEB, Nemours, France) a été fixé un antisérum de chèvre anti-IgG de souris (Cappel) dilué dans une solution saline tamponnée au borate (BBS) 0,2 M, pH 8,2 à une concentration de 2 µg/ml.

Après une nuit d'incubation à 4°C, les plaques ont été lavées dans une solution tampon contenant de l'albumine de sérum de boeuf (BSA), 1 % de PBS, le tout à température ambiante pendant une heure.

Les IgG éluées de reins ont alors été ajoutées. Après incubation des plaques pendant une heure à 37°C et 3 lavages de ces plaques avec du TWEEN 20-PBS, 100µl d'antisérum de chèvre anti-IgG de souris marqué à la β-galactosidase dilué au 1 : 500, ont été ajoutés et les plaques ont été incubées pendant une heure à 37°C.

Les plaques ont alors été lavées 3 fois avec du TWEEN 20-PBS, avant addition de 100µl d'ornitrophenyl-β-Dgalactopyranoside (ONPG) (Sigma) en tant que substrat pendant 30 mn à 37°C.
- La capacité de fixation des anticorps de sérums de souris lupiques à l'ADN a été mesurée par la méthode radioimmunologique en phase solide décrite dans J. Immunol. (1980) 125, 2805-2808.
- la réactivité immunologique des protéines membranaires contenues dans le "surnageant élastase" avec des IgG de rein a été mesurée par la technique d'immunotransfert décrite plus haut.

2) Resultats

- Quantification des IgG de reins élués

Les quantitiés d'IgG éluées et dosées dans le test ELISA ci-dessus décrites, sont respectivement de l'ordre de 185 µg pour les souris MRL/lpr et de 1,8 µg pour les souris BALB/c.

- Capacité de fixation à l'ADN des sérums de reins

Les sérums des souris MRL/lpr et B/W se lient à l'ADNdb par test radioimmunologique en phase solide (tableau III).

Les souris BALB/c ne se lient pas à l'ADNdb (tableau III).

- Analyse des immunotransferts des IgG éluées à partir de souris MRL/lpr

Au moins 5 bandes majeures de 34 KD, 33 KD, 17 KD, 16 KD et 14 KD ont été détectées dans le "surnageant élastase" avec les IgG éluées de souris MRL/lpr en utilisant un extrait de cellules IW 32.

## TABLEAU III

Capacité de fixation d'ADN mesuré par un essai radio-immunologique en phase solide

--------------------------------------------------------

| Source de sérum | fixation exprimée en coups par minute (cpm). |
|---|---|
| Sérum de souris MRL/lpr dilué au 1 : 100 | 3018 ± 325 |
| Sérum de souris B/W dilué au 1 : 100 | 3408 ± 270 |
| Sérum normal dilué au 1 : 100 | 527 ± 74 |
| Surnageant de AcM PME77 | 5378 ± 285 |
| Surnageant d'AcM anti-Golgi | 214 ± 73 |

--------------------------------------------------------

De manière à tester la réaction croisée existante entre ces 5 polypeptides et l'ADNdb, les IgG éluées ont été préincubées avec 1 mg/ml d'ADNdb.

L'intensité de toutes les bandes protéiques ont alors été fortement réduites.

En revanche aucune bande n'a été détectée dans les mêmes conditions lorsque les IgG de souris BALB/c ont été utilisées comme témoins de contrôle.

Ces résultats démontrent que les IgG éluées de souris MRL/lpr réagissent spécifiquement avec la (les) protéine(s) de surface, ayant un épitope commun avec l'ADNdb.

- Analyse d'immunorépliques de sérums de souris autoimmunes

Les mêmes bandes majeures de 34 KD, 33 KD, 17 KD, 16 KD et 14 KD ont été détectées avec l'AcM PME77 et les sérums dilués au 1 : 50 des souris MRL/lpr et B/W, en utilisant le "surnageant d'élastase" de cellules IW 32.

Toutefois, probablement en raison d'une extrême sensibilité de la (les) protéine(s) aux protéases, quelques autres bandes ont été détectées dans certaines expériences, en plus des bandes majeures en fonction des conditions expérimentales.

Dans tous les cas, une forte réaction a été observée.

Ces bandes ont été fortement réduites lorsque les sérums de souris MRL/lpr et B/W, dilués au 1 : 50, ont été préincubés avec 1 mg/ml d'ADNdb. Aucune bande n'a été détectée par mise en contact de l'AcM PME77 avec des sérums de souris BALB/c utilisés en tant que témoins.

Ces résultats démontrent que les anticorps dirigés contre les polypeptides de surface cellulaire sont présents en grande quantité dans les sérums de souris MRL/lpr et B/W.

III. Mise en évidence de la présence d'anticorps spécifiquement reconnus par les polypeptides de surface cellulaire dans les sérums d'individus atteints de LED

1) Malades et méthodes

- Malades

Les échantillons de sérums provenant du groupe de 25 individus atteints de LED mentionné dans le préambule de cette description, et d'autres groupes comprenant respectivement 10 malades atteints de polyarthrite rhumatoïde, 4 malades atteints de sclérodermie, 2 malades présentant le syndrome de Goujerot Sjogren et de 10 individus normaux, ont été étudiés.

Les produits des réactions immunologiques entre ces sérums et le "surnageant élastase" ont été analysés par immunotransfert comme précédemment décrit.

- Capacité de liaison à l'ADN

Tous les sérums ont été testés en ce qui concerne la présence d'anticorps anti-ADN par la méthode de Farr (N. Engl. J. Med. (1969) 281, 701-705).

Deux d'entre eux ont également été analysés par test radioimmunologique en phase solide et par le test utilisant Crithidia lucilae (J. Immunol. (1980) 125, 2805-2808).

2) Résultats

- Analyse des immuntransferts des sérums LED

Des bandes correspondant aux polypeptides 34 KD, 33 KD, 17 KD, 16 KD et 14 KD ont été détectées dans les 25 sérums LED humains testés et dilués au 1 : 20 en utilisant le "surnageant d'élastase" comme antigène.

La totalité des bandes n'a pas toujours été observée chez certains des malades. De plus, quelques autres bandes ont été détectées en quelques occasions en plus des bandes majeures, peut-être en raison de l'extrême sensibilité des polypeptides à l'action des protéases en fonction des conditions expérimentales.

La plupart des sérums LED présentaient une forte réaction (spécialement pour les polypeptides de 17 KD), et quelques autres en présentaient une faible.

Il est cependant essentiel d'observer que des bandes ont été nettement détectées chez tous les malades atteints de LED, selon les normes définies dans la classification révisée de l'American Rhumatoïd Association, y compris chez les deux malades chez lesquels les tests de détection des anticorps anti-ADN (test de Farr) et les tests radioimmunologiques en phase solide et Crithida lucilae, (immunofluorescence) avaient conduits à des résultats négatifs.

Quand les sérums de malades LED dilués au 1 : 20 ont été préincubés avec de l'ADNdb (1 mg/ml), ces bandes ont été fortement réduites.

Ces résultats démontrent clairement la présence d'anticorps contre ces polypeptides de surface cellulaires, qui ont un épitope en commun avec l'ADNdb, dans les sérums LED testés.

Réciproquement, aucune réaction immunologique n'a été observée avec les 10 sérums humains normaux, et les sérums des autres groupes de malades atteints de polyarthrite rhumatoïde, de sclérodermie et du syndrome de Goujerot Sjögren.

- Corrélation avec les niveaux d'anticorps anti-ADNdb

Les anticorps anti-ADNdb ont été mesurés par la technique de Farr pour cette étude.

Aucune corrélation évidente n'a été trouvée entre le titre en anticorps anti-ADNdb par la méthode de Farr et celui en anticorps anti-polypeptides (ou anti-LAMP) par analyse d'immunotransfert.

Toutefois, il faut rappeler que cette dernière technique est seulement semi-quantitative mais très sensible.

IV. Des cellules de rate de souris lupiques MRL/lpr/lpr et B/W ont été traitées respectivement à l'élastase, la papaïne et la trypsine, puis le surnageant élastase, le surnageant papaïne et le surnageant trypsine ont été récupérés. Dans ces conditions, en immunotransfert (immunoblot), le PME 77 reconnaît un polypeptide majeur de 55 KD présent dans le surnageant élastase, le surnageant papaïne et le surnageant trypsine.

En revanche, en traitant des cellules de rate de souris normales BALB/C ou CBA/ca à l'élastase, la papaïne et la trypsine, le PME77 détecte avec le surnageant élastase les polypeptides 34 KD, 33 KD, 17 KD, 16 KD et 14 KD, mais ne détecte aucun polypeptide avec le surnageant papaïne et le surnageant trypsine, du fait de la grande sensibilité de cette protéine membranaire (LAMP), présente à la surface des cellules normales, aux enzymes protéolytiques.

L'ensemble de ces données démontre la résistance de la LAMP aux protéases dans les cellules de souris lupiques B/W et MRL/lpr/lpr. Cette résistance s'explique par un changement, soit dans la structure de la LAMP, soit dans son environnement à la membrane.

Il a été démontré, par immunoblot, que des anticorps contre ce polypeptide 55 KD sont présents dans le sérum des souris lupiques B/W et MRL/lpr/lpr.

Ce polypeptide 55 KD, isolé par chromatographie d'affinité dans les conditions sus-indiquées est reconnu en immunoblot par les 4 sérums de patients atteints de lupus érythémateux disséminé. Ce polypeptide peut donc être utilisé aussi pour le diagnostic invitro, sensible et spécifique, du LED.

V. 29 sérums positifs sur 30 de patients atteints de lupus en évolution ont des anticorps contre les polypeptides membranaires (P 34, P 33, P 17, P 16, P 14).

Par ailleurs 5 patients atteints de lupus ont été testés en poussée et en rémission (retour clinique à la normale) : en poussée, les malades avaient des anticorps contre les polypeptides membranaires qui disparaissaient à l'état normal.

10 autres sérums de patients atteints de lupus en rémission n'avaient pas d'anticorps contre les polypeptides membranaires.

Les recherches d'anticorps contre ces polypeptides peuvent donc être utilisés comme marqueurs pour la surveillance évolutive de la maladie lupique. A ce titre, ces protéines ou polypeptides constituent donc des marqueurs meil leurs que les anticorps anti-ADN. Ceux-ci sont en effet souvent détectés aussi bien chez les malades en rémission que chez ceux en poussée.

L'étude de cellules épithéliales de rat utilisant l'anticorps monoclonal PME 77 a montré en microscopie électronique que la protéine LAMP était présente dans les vésicules de Clathrin (coated pits) transportant des

0 252 787

molécules de surface, en particulier des récepteurs membranaires, à l'intérieur des cellules.

La LAMP est modifiée non seulement à la surface des lymphocytes de souris, mais aussi à la surface des cellules glomérulaires de souris lupiques.

## Revendications

1. Composition de polypeptides caractérisée
- en ce que qu'ils sont spécifiquement reconnus par l'AcM PME77
- en ce que ces polypeptides sont reconnus par des sérums de souris lupiques et de patients humains affectés par le lupus érythémateux disséminé (LED) et non reconnus par des sérums obtenus à partir de personnes saines ou de souris saines,
- en ce que ces polypeptides sont immunogènes chez le lapin et induisent des anticorps qui de reconnaissent pas l'ADN double brin,
- en ce que ces polypeptides sont reconnus par des immunoglobulines rénales de souris lupiques et ne sont pas reconnus par des immunoglobulines rénales de souris non lupiques.

2. Composition de polypeptides appartenant à une ou plusieurs espèces moléculaires distinctes et caractérisée
- en ce que qu'ils sont présents en une concentration suffisamment élevée pour autoriser leur fixation sur l'AcM PME77 lui-même immobilisé sur un support insolubles des opérations de chromatographie d'affinité,
- en ce que ces polypeptides sont reconnus par des sérums de souris lupiques et de patients humains affectés par le lupus érythémateux disséminé (LED) et non reconnus par des sérums obtenus à partir de personnes saines ou de souris saines,
- en ce que ces polypeptides sont immunogènes chez le lapin et induisent des anticorps qui ne reconnaissent pas l'ADN double brin humain ou de souris,
- en ce que ces peptides sont reconnus par des immunoglobulines rénales de souris lupiques et ne sont pas reconnus par des immunoglobulines rénales de souris non lupiques.

3. Composition de polypeptides selon la revendication 1 ou 2, caractérisée en ce que ces polypeptides ont un épitope commun avec l'ADN double brin.

4. Composition selon la revendication 1 ou 2, caractérisée en ce que ces polypeptides appartiennent à l'une au moins des espèces moléculaires respectivement caractérisées par des poids moléculaires d'environ 50-55 KD, 43 KD, 34 KD, 33 KD, 17 KD, 16 KD, et 14 KD, ou sont des produits de dégradation d'une protéine de poids moléculaire élevé et reconnue par l'AcM PME77.

5. Composition selon la revendication 1 ou 2, caractérisée en ce qu'elle est reconnue par un anticorps de lapin obtenu par immunisation de lapins avec une composition de peptides selon la revendication 4 appartenant aux trois espèces moléculaires de poids moléculaires 17 KD, 16 KD, et 14 KD.

6. Composition selon l'une des quelconques revendications 1 à 5, caractérisée en ce que les polypeptides sont fixables en quasi-totalité sur l'AcM PME77 immobilisé sur support de chromatographie d'affinité.

7. Procédé de détection in vitro d'anticorps corrélable au lupus erythémateux disséminé (LED) dans un tissu ou fluide biologique susceptible de les contenir, comprenant la mise en contact d'une composition conforme à l'une quelconque des revendications 1 à 6 avec ce tissu ou fluide biologique dans des conditions permettant une réaction immunologique in vitro entre lesdits polypeptides et les anticorps éventuellement présents dans le tissu ou fluide biologique, et la détection in vitro du complexe antigène-anticorps éventuellement formé.

8. Procédé de détection in vitro d'anticorps corrélable au lupus erythémateux disséminé (LED) dans un tissu ou fluide biologique susceptible de les contenir, comprenant la mise en contact d'un polypeptide reconnu par l'AcM PME77 avec ce tissu ou fluide biologique dans des conditions permettant une réaction immunologique in vitro entre lesdits polypeptides et les anticorps éventuellement présents dans le tissu ou fluide biologique, et la détection in vitro du complexe antigène-anticorps éventuellement formé.

9. Procédé selon les revendications 7 ou 8 appliqué au diagnostic in vitro du LED et en ce que le milieu biologique étudié est un sérum humain.

10. Coffret de diagnostic ou kit pour la détection in vitro d'anticorps caractéristiques du LED dans un tissu ou fluide biologique comprenant
- au moins un polypeptide reconnu par l'AcM PME77, notamment une composition selon l'une quelconque des revendications 1 à 6
- les réactifs pour la constitution du milieu propice à la réalisation de la réaction immunologique,
- des réactifs permettant la détection du complexe antigènes-anticorps produit par la réaction immonologique,
- le cas échéant un tissu ou fluide biologique de référence dépourvu d'anticorps reconnu par la composition selon l'une quelconque des revendications 1 à 6,
- le cas échéant aussi, un échantillon de comparaison contenant lesdits anticorps.

11. Coffret de diagnostic ou kit selon la revendication 10 caractérisé en ce que les réactifs permettant la

14

détection du complexe antigènes-anticorps portent un marqueur ou sont susceptibles d'être reconnus à leur tour par un réactif marqué.

12. Anticorps contre les polypeptides caractéristiques de la composition selon l'une quelconque des revendications 1 à 6.

13. Procédé d'obtention d'une composition selon l'une quelconque des revendications 1 à 6, caractérisé par la mise en incubation de cellules humaines, de souris ou de rats dans une solution d'élastase dosée de façon à permettre une extraction sélective desdits polypeptides à partir de la surface desdites cellules, par la récupération du surnageant contenant lesdits polypeptides reconnus par les anticorps monoclonaux PME77.

14. Procédé selon la revendication 13, caractérisé en ce que l'incubation des cellules est réalisée dans dans une solution tamponnée à un pH compris entre 8 et 8,5, notamment à pH 8,2 en présence d'élastase à une concentration finale de 5 à 20 µg/ml notamment de 10 µg/ml pendant une durée de 5 à 15 minutes, notamment 7 mn, à une température de 4°C.

15. Procédé selon la revendication 13 ou 14, caractérisé par une purification supplémentaire comprenant la mise en contact du susdit surnageant avec des anticorps monoclonaux PME77 ou des anticorps conformes à la revendication 12 immobilisés sur un support de chromatographie d'affinité, et la récupération des polypeptides retenus par mise en contact du support de chromatographie d'affinité avec une solution de dissociation du complexe antigène-anticorps formé.

## DOCUMENTS CONSIDERES COMME PERTINENTS

| Categorie | Citation du document avec indication. en cas de besoin. des parties pertinentes | Revendication concernee | CLASSEMENT DE LA DEMANDE (Int Cl 4) |
|---|---|---|---|
| X,D | PROC. NATL. ACAD. SCI. USA, vol. 81, juin 1984, pages 3843-3845; L. JACOB et al: "A monoclonal anti-DNA antibody also binds to cell-surface protein(s)" * Page 3843, colonne de gauche, lignes 7-14; page 3844, colonne de droite, lignes 13-26,42 - page 3845, colonne de droite, ligne 14 * | 1-4,6-12 | G 01 N   33/68 G 01 N   33/564 C 07 K   15/06 C 07 K    3/02 |
| | --- | | |
| X | J. CLIN. INVEST., vol. 75, janvier 1985, pages 315-317, The American Society for Clinical Investigation, Inc., US; L. JACOB et al.: "Binding of a monoclonal anti-DNA autoantibody to identical protein(s) present at the surface of several human cell types involved in lupus pathogenesis" * Page 315, colonne de gauche, lignes 8-19; page 316, colonne de gauche - colonne de droite, "Results" * | 1-4,6, 12 | |

**DOMAINES TECHNIQUES RECHERCHES (Int. Cl.4)**

A 61 K
G 01 N

---      -/-

Le présent rapport de recherche a été établi pour toutes les revendications

| Lieu de la recherche | Date d'achèvement de la recherche | Examinateur |
|---|---|---|
| LA HAYE | 24-09-1987 | CHARLES D.J.P.I.G. |

| | DOCUMENTS CONSIDERES COMME PERTINENTS | | Page 2 |
|---|---|---|---|
| Categorie | Citation du document avec indication, en cas de besoin, des parties pertinentes | Revendication concernée | CLASSEMENT DE LA DEMANDE (Int Cl 4) |
| X,P | PROC. NATL. ACAD. SCI. USA, vol. 83, septembre 1986, pages 6970-6974; L. JACOB et al.: "Human systemic lupus erythematosus sera contain antibodies against cell-surface protein(s) that share(s) epitope(s) with DNA" * En entier * | 1-6,12 -15 | |
| X,P | CHEMICAL ABSTRACTS, vol. 106, no. 19, 11 mai 1987, page 533, résumé no. 154552a, Columbus, Ohio, US; L. JACOB et al.: "Altered cell-surface protein(s), crossreactive with DNA, on spleen cells of autoimmune lupic mice", & PROC. NATL. ACAD. SCI. USA 1987, 84(5), 1361-3 * En entier * | 1-6,12 -15 | |
| X,P | CHEMICAL ABSTRACTS, vol. 107, no. 1, 6 juillet 1987, page 521, résumé no. 5530k, Columbus, Ohio, US; L. JACOB et al.: "Presence of antibodies against a cell-surface protein, cross-reactive with DNA, in systemic lupus erythematosus: a marker of the disease", & PROC. NATL. ACAD. SCI. USA 1987, 84(9), 2956-9 * En entier * --- -/- | 1,7-12 | DOMAINES TECHNIQUES RECHERCHES (Int. Cl.4) |

Le present rapport de recherche a été etabli pour toutes les revendications

| Lieu de la recherche | Date d'achèvement de la recherche | Examinateur |
|---|---|---|
| LA HAYE | 24-09-1987 | CHARLES D.J.P.I.G. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié a la date de dépôt ou après cette date
D : cité dans la demande
L : cité pour d'autres raisons

& : membre de la même famille, document correspondant

OEB Form 1503 03 82

| | **DOCUMENTS CONSIDERES COMME PERTINENTS** | | Page 3 |
|---|---|---|---|
| Categorie | Citation du document avec indication, en cas de besoin, des parties pertinentes | Revendication concernée | CLASSEMENT DE LA DEMANDE (Int Cl 4) |
| A | CHEMICAL ABSTRACTS, vol. 101, no. 11, 10 septembre 1984, page 464, résumé no. 88587j, Columbus, Ohio, US; J. RAUCH et al.: "Monoclonal anti-cardiolipin antibodies binding to DNA", & EUR. J. IMMUNOL. 1984, 14(6), 529-34 * Résumé * | 1,3 | |
| A | GB-A-2 146 338 (YEDA RESEARCH AND DEVELOPMENT CO., LTD) * Page 1, lignes 5-12,44-49,57-60; revendications 6,7 * | 1,2,13 | |
| A | MONOCLONAL ANTIBODIES AND T CELL PRODUCTS, 1982, pages 1,6,7, D.H. Katz, M.D., CRC Press, Inc., Boca Raton, Florida, US; F. T. LIU et al.: "Monoclonal antibodies (mAbs) as useful research and diagnostic probes" * Spécialement pages 6,7 * | 13,15 | DOMAINES TECHNIQUES RECHERCHES (Int. Cl.4) |

Le présent rapport de recherche a été établi pour toutes les revendications

| Lieu de la recherche | Date d'achèvement de la recherche | Examinateur |
|---|---|---|
| LA HAYE | 24-09-1987 | CHARLES D.J.P.I.G. |